# DEMANDE DE BREVET EUROPEEN

(11) **EP 0 788 835 A1**
(43) Date de publication de la demande: **13.08.1997**
(21) Numéro de dépôt: 97400254.5
(22) Date de dépôt: 05.02.1997
(51) Int. Cl.: B01J 29/06

(54) **Catalyseur à base de zéolithe de type structural mazzite modifiée et son utilisation en dismutation et/ou transalkylation d'hydrocarbures alkylaromatiques**

(30) Priorité: 09.02.1996 FR 9601604
(71) Demandeur: INSTITUT FRANCAIS DU PETROLE, 92502 Rueil-Malmaison (FR)
(72) Inventeur: Benazzi, Eric, 78360 Montesson (FR); Alario, Fabio, 92200 Neuilly sur Seine (FR)

(57) **Abrégé**

L'invention concerne un catalyseur comprenant au moins une matrice et au moins une zéolithe de type structural mazzite, de préférence la zéolithe oméga, comprenant du silicium et de l'aluminium, au moins en partie, de préférence pratiquement totalement, sous forme acide, laditte zéolithe ayant été préparée par désalumination de la charpente, par au moins un traitement par au moins une solution d'un fluorosilicate d'un cation à raison de 0,05 à 5 moles par mole d'aluminium contenue dans la zéolithe sèche, ledit catalyseur comprenant éventuellement au moins un élément choisi parmi les groupes IB et VIII de la classification périodique des éléments. L'invention concerne aussi l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation de toluène et de triméthylbenzènes pour produire des xylènes.

## Description

L'invention concerne un catalyseur comprenant au moins une matrice et au moins une zéolithe de type structural mazzite, de préférence la zéolithe oméga, comprenant du silicium et de l'aluminium, au moins en partie, de préférence pratiquement totalement, sous forme acide, laditte zéolithe ayant été préparée par désalumination de la charpente, par au moins un traitement par au moins une solution d'un fluorosilicate d'un cation à raison de 0,05 à 5 moles par mole d'aluminium contenue dans la zéolithe sèche, ledit catalyseur comprenant éventuellement au moins un élément choisi parmi les groupe IB et VIII de la classification périodique des éléments. L'invention concerne aussi l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation du toluène et de triméthylbenzènes pour produire des xylènes.

De nombreux catalyseurs de dismutation et de transalkylation à base de mordénite ont déjà été décrits dans l'art antérieur. La mordénite utilisée possède un réseau microporeux monodimensionnel dont le diamètre des pores est de 7 x 6,5 Å (1 Å = 1 Angström = 1.10⁻¹⁰ m) ("Atlas of Zeolites Structure Types", W.M. Meier et D.H. Olson, 3ème Edition, 1992). Il en est ainsi dans le brevet US-A-3.506.731 où une mordénite sous forme hydrogène est utilisée ainsi que dans la demande de brevet français FR-A-2.367.533. Il en est encore ainsi dans le brevet US-A-3.281.483 qui fait mention de mordénites échangées essentiellement avec des ions argent ou nickel, ou bien dans le brevet US-A-3.780.121 qui fait état d'une mordénite échangée avec des métaux du groupe lB de la classification périodique des éléments et qui est caractérisée par un rapport atomique Si/Al compris entre 6 et 40, ou bien encore dans le brevet US-A-3.629.351 qui concerne également une mordénite contenant des ions des métaux du groupe IB, VA, VIA, IIA et VIII de la classification périodique des éléments.

La mazzite présente dans le catalyseur selon l'invention peut éventuellement avoir subi des modifications post-synthèse comme par exemple une désalumination par au moins un traitement par au moins une solution comprenant au moins un sel de fluorosilicate tel que l'hexafluorosilicate d'ammonium, comme cela est décrit dans le brevet US-A-4.503.023, qui décrit un tel traitement pour de nombreuses zéolithes et permet d'atteindre, selon la nature de la zéolithe, un taux de désalumination au moins égal à 30 %, ou bien dans la demande de brevet européen EP-A-0.573.347, qui concerne un catalyseur à base de mordénite ainsi modifiée, la concentration du sel étant particulièrement faible par rapport à celle utilisée dans le brevet précédent, et son utilisation en isomérisation de composés alkylaromatiques comportant 8 atomes de carbone par molécule.

De façon surprenante, un catalyseur comprenant au moins une matrice et au moins une zéolithe de type structural mazzite, au moins en partie acide, ayant été préparée par au moins un traitement par au moins une solution d'un fluorosilicate d'un cation à raison de 0,05 à 5 moles par mole d'aluminium contenue dans la zéolithe sèche, de préférence entre 0,06 et 4 moles par mole d'aluminium contenue dans la zéolithe sèche, ledit catalyseur comprenant éventuellement au moins un élément choisi parmi les groupe IB et VIII de la classification périodique des éléments, conduit à des performances catalytiques, et en particulier des sélectivités, améliorées dans les réactions de dismutation d'hydrocarbures alkylaromatiques tel que le toluène, et/ou de transalkylation d'hydrocarbures alkylaromatiques tels que le toluène et les triméthylbenzènes, par rapport aux catalyseurs de l'art antérieur.

L'invention concerne un catalyseur comportant au moins une matrice (ou liant) et au moins une zéolithe de type structural mazzite, de préférence la zéolithe oméga, au moins en partie, de préférence pratiquement totalement, sous forme acide, comprenant du silicium et de l'aluminium, ayant été préparée par au moins un traitement par au moins une solution d'un fluorosilicate d'un cation à raison de 0,05 à 5, de préférence de 0,06 à 4 moles par mole d'aluminium contenue dans la zéolithe sèche, ledit catalyseur comprenant éventuellement au moins un élément choisi dans l'ensemble formé par les groupes IB et VIII de la classification périodique des éléments.

La matrice est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium et les silice-alumines, de préférence parmi les éléments du groupe formé par les alumines et les argiles.

La zéolithe de type structural mazzite selon l'invention est généralement choisie dans le groupe formé par la zéolithe oméga, la mazzite, la zéolithe LZ-202 ou la zéolithe ZSM-4, de préférence la zéolithe oméga, dont le diamètre des pores principaux est d'environ 7,4 Å et qui posséde un réseau microporeux monodimensionnel ("Atlas of Zeolites Structure Types", W.M. Meier et D.H. Olson, 3ème Edition, 1992).

L'étape de désamunination de la charpente par traitement par au moins une solution comprenant au moins un sel de fluorosilicate peut éventuellement avoir lieu après une étape de d'extraction plus classique telle que l'étape de désalumination décrite dans le brevet US-A-4.780.436.

Le catalyseur selon l'invention contient généralement de 10 à 99%, et de préférence de 20 à 95%, de zéolithe de type structural mazzite, de préférence de zéolithe oméga, au moins en partie, de préférence pratiquement totalement, sous forme acide. Dans le cas où le catalyseur de la présente invention contient au moins un élément choisi dans l'ensemble formé par les groupes IB et VIII de la classification périodique des éléments, la teneur pondérale du(des)dit(s) élément(s) est généralement comprise entre 0,01 et 10%, de préférence entre 0,05 et 7%, et de façon encore plus préférée entre 0,10 et 5%. Le complément à 100 % poids consiste généralement en la part de matrice dans le catalyseur.

L'invention concerne aussi la préparation de ladite zéolithe de type structural mazzite et dudit catalyseur.

La zéolithe de type structural mazzite comprise dans le catalyseur utilisé selon l'invention, comprenant du silicium et de l'aluminium, et a un rapport Si/Al atomique global compris entre 3,2 et 100, de préférence entre 6 et 80, et de manière encore plus préférée entre 8 et 60, ainsi qu'une teneur en sodium par rapport au poids de zéolithe sèche inférieure à 0,6 % poids, de préférence inférieure à 0,1 % poids

Pour préparer la zéolithe de type structural mazzite selon l'invention, on opère une désalumination d'une zéolithe de type structural mazzite brute de synthèse par toute méthode connue de l'homme du métier, en particulier selon le mode opératoire décrit dans le brevet US-A-4.780.436 lorsque T est l'aluminium, c'est-à-dire que l'on procède à une étape de calcination sous flux d'air sec, qui a pour but d'éliminer le structurant organique occlus dans la microporosité de la zéolithe, à au moins un échange ionique par au moins une solution de NH₄NO₃, de manière à éliminer pratiquement tout cation alcalin, en particulier le sodium, présent en position cationique dans la zéolithe, puis à au moins un cycle de désalumination de la charpente, comportant au moins une calcination en présence de vapeur d'eau, à une température généralement comprise entre 550 et 850 °C, suivie d'au moins une attaque acide.

Le cycle de désalumination de la charpente, comportant au moins une étape de calcination sous vapeur d'eau et au moins une étape d'attaque en milieu acide de la zéolithe de type structural mazzite, peut être répété autant de fois qu'il est nécessaire pour obtenir la zéolithe de type structural mazzite désaluminée possédant les caractéristiques désirées. De même, suite au traitement de calcination sous vapeur d'eau, plusieurs attaques acides successives, avec des solutions en acide de concentration différentes, peuvent être opérées.

La zéolithe est soumise à au moins un traitement par au moins une solution diluée de fluorosilicate d'un cation, de préférence par au moins une solution diluée d'hexafluorosilicate d'ammonium, qui comprend généralement entre 0,05 et 5, de préférence entre 0,06 et 4 moles par mole d'aluminium contenue dans la zéolithe sèche. On obtient ainsi un taux de désalumination global inférieur à 5%, soit généralement compris entre 0 (borne exclue) et 5 %, pour la zéolithe ainsi traitée.

Le fluorosilicate utilisé en tant qu'agent de désalumination de la charpente zéolithique et en tant que source de silicium, permettant ainsi la réinsertion d'atomes de silicium au sein du réseau cristallin de la zéolithe à la place des atomes d'aluminium extraits, est choisi parmi les sels de fluorosilicate possédant la formule suivante: M_{2/x}SiF₆ où M est un cation métallique ou non métallique possédant la valence x. Le cation M peut donc être choisi dans le groupe formé par NH₄⁺, des alkyls ammonium, K⁺, Na⁺, Li⁺, Ba²⁺, Mg²⁺, Cd²⁺, Cu⁺, Cu²⁺ Ca²⁺, Cs⁺, Fe²⁺, Co²⁺, Pb²⁺, Mn²⁺, Rb⁺, Ag⁺, Sr²⁺, Zn²⁺, Tl⁺ et H⁺.

De préférence, l'hexafluorosilicate d'ammonium est utilisé, car il conduit à la formation de sel d'ammonium (NH₄)₃AlF₆ soluble dans l'eau qui peut être ainsi aisément éliminé. En général, la température de traitement de la zéolithe de type structural mazzite par l'hexafluorosilicate d'ammonium est comprise entre 20 et 100°C, et de préférence entre 50 et 100 °C. Les quantités d'hexafluorosilicate d'ammonium utilisé sont calculées par rapport à une zéolithe séchée sous flux d'air, à 450° C pendant 4 heures. Le traitement de la zéolithe de type structural mazzite s'effectue en présence d'acétate d'ammonium qui permet de tamponner le pH du milieu réactionnel à des valeurs, comprises entre 4 et 8, et de préférence entre 5,5 et 7, valeurs de pH pour lesquelles la zéolithe ne subit pas de destruction de la charpente par attaque acide directe.

Après l'étape d'addition de la solution d'hexafluorosilicate d'ammonium à la suspension de zéolithe de type structural mazzite dans une solution d'acétate d'ammonium, le mélange réactionnel est laissé, sous agitation vigoureuse, à la température désirée pendant une période comprise entre 30 minutes et 48 heures, mais de préférence comprise entre 1 et 2 heures.

La zéolithe de type structural mazzite est ensuite filtrée à la température de la réaction et abondamment lavée à l'eau bouillante. Le volume d'eau bouillante utilisée pour effectuer ces lavages correspond à un v/p = 150 ml/g, (le rapport v/p est le rapport volume d'eau bouillante sur quantité de zéolithe sèche traitée).

Après ce traitement, la zéolithe de type structural mazzite modifiée subit un traitement thermique destinée à décomposer les cations ammonium présents au sein du réseau et à obtenir ainsi la forme acide de la zéolithe.

Ainsi, la zéolithe de type structural mazzite obtenue possède une teneur pondérale en sodium par rapport au poids de zéolithe oméga sèche inférieure à 2000 ppm, un rapport atomique Si/Al global compris entre 3,2 et 100 et, de préférence entre 6 et 80 et de manière encore plus préférée entre 8 et 60.

Les caractéristiques de la zéolithe de type structural mazzite peuvent être mesurées par les méthodes suivantes :
- le rapport atomique Si/Al est déterminé par fluorescence X et par résonance magnétique nucléaire du silicium 29,
- la teneur en sodium est déterminé par absorption atomique,
- le volume de maille élémentaire et la cristallinité sont déterminés par diffraction X, l'échantillon de zéolithe oméga étant préparé comme dans le mode opératoire de la norme ASTM D3942 80 établie pour la faujasite.

La préparation du catalyseur être effectuée selon toute méthode connue de l'homme du métier. En général, elle est obtenue par mélange de la matrice et de la zéolithe puis par mise en forme. L'élément éventuel de l'ensemble formé par les groupe lB et VIII de la classification périodique des éléments peut être intoduit soit avant la mise en forme, ou bien lors du mélange, ou bien sur la zéolithe elle même avant de la mélanger, soit, de préférence, après la mise en forme. La mise en forme est généralement suivie d'une calcination, généralement à une température comprise entre 250 et 600 °C. L'élément éventuel de l'ensemble formé par les groupe IB et VIII de la classification périodique des éléments peut être intoduit après ladite calcination. Dans tous les cas, ledit élément est généralement déposé au choix soit, de préférence, pratiquement totalement sur la zéolithe, soit pratiquement totalement sur la matrice, soit en partie sur la zéolithe et en partie sur la matrice, ce choix s'effectuant, de la manière qui est connue de l'homme du métier, par les paramètres utilisés lors dudit dépôt, comme par exemple la nature du précurseur choisi pour effectuer ledit dépôt.

L'élément des groupes IB ou VIII, de préférence choisi dans le groupe formé par Ag, Ni et Pt, et de façon encore plus préférée Ni, peut également éventuellement être déposé sur le mélange zéolithe-matrice préalablement mis en forme par tout procédé connu de l'homme de l'art. Un tel dépôt est généralement effectué par la technique d'imprégnation à sec, d'échange(s) ionique(s) ou de coprécipitation. Dans le cas de l'échange ionique à partir de précurseurs à base d'argent, de nickel ou de platine, on utilise habituellement des sels d'argent tels que les chlorures ou les nitrates, un complexe tétramine du platine, ou des sels de nickel tels que les chlorures, les nitrates, les acétates ou les formiates. Cette technique d'échange cationique peut également être utilisée pour déposer directement le métal sur la poudre de zéolithe, avant son mélange éventuel avec une matrice.

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

Par exemple, une des méthodes préférées de préparation du catalyseur selon l'invention consiste à malaxer la zéolithe dans un gel humide de matrice (obtenu généralement par mélange d'au moins un acide et d'une poudre de matrice), par exemple d'alumine, pendant une durée nécéssaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques minutes à 100 °C en étuve et après calcination, par exemple pendant 2 heures à 400 °C, l'élément éventuel, par exemple le nickel, peut être déposé, par exemple par échange ionique, ledit dépôt étant suivi d'une calcination finale, par exemple pendant 2 heures à 400 °C.

La mise en forme du catalyseur selon l'invention est généralement telle que le catalyseur est de préférence sous forme de pastilles, d'aggrégats, d'extrudés ou de billes, en vue de son utilisation.

La préparation du catalyseur se termine généralement par une calcination, dite calcination finale, habituellement à une température comprise entre 250 et 600 °C, de préférence précédée d'un séchage, par exemple à l'étuve, à une température généralement comprise entre la température ambiante et 250 °C, de préférence entre 40 et 200 °C. Ladite étape de séchage est de préférence menée pendant la montée en température nécessaire pour effectuer ladite calcination.

L'invention concerne aussi l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation d'hydrocarbures alkylaromatiques généralement en C₉⁺ (c'est-à-dire à au moins 9 atomes de carbone par molécule), telle que la transalkylation et/ou la dismutation du toluène et/ou d'alkylaromatiques C₉⁺ pour produire des xylènes. La charge d'un tel procédé peut comprendre de 0 à 100 % d'alkylaromatiques en C₉⁺ et de 0 à 100 % de toluène.

Les conditions opératoires sont généralement les suivantes : une température comprise entre 250 et 600°C et de préférence entre 330 et 500°C ; une pression comprise entre 10 et 60 bar et de préférence entre 20 et 45 bar; une vitesse spatiale d'alimentation, exprimée en kilogramme de charge introduite par kilogramme de catalyseur et par heure, comprise entre 0,1 et 10 et de préférence entre 0,5 et 4 ; un rapport molaire hydrogène sur hydrocarbures compris entre 2 et 20 et de préférence entre 3 et 12.

Les exemples suivants illustrent l'invention sans toutefois en limiter la portée.

### Exemple 1 : Préparation de la zéolithe Ω1 selon l'invention

La matière première utilisée est une zéolithe oméga, qui possède un rapport atomique Si/Al global égal à 3,2, une teneur pondérale en sodium par rapport au poids en zéolithe oméga sèche d'environ 5,3 %, un volume de maille élémentaire de 2,196 nm³, et un volume poreux, à l'azote, mesuré à -196°C et à P/Pₒ=0,19 de 0,125 cm³ liquide par gramme.

Cette zéolithe oméga subit tout d'abord une calcination dite sèche à 550°C sous flux d'air et d'azote durant 6 heures. Puis, le solide obtenu est soumis à trois échanges ioniques dans une solution de NH₄NO₃ 10N, à environ 100°C pendant 4 heures pour chaque échange. La zéolithe oméga est alors soumise à un traitement hydrothermique, en présence de 50% de vapeur d'eau à 625°C, durant 4 heures. La zéolithe subit alors une attaque acide, à l'aide d'acide nitrique 1,5N, à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10).

A l'issue de ces traitements, la zéolithe oméga sous forme H a un rapport Si/Al atomique global égal à 11,3, un rapport Si/Al de charpente, déterminé par RMN du ²⁹Si de 12, un rapport Si/AI de la surface externe des cristaux de zéolithe oméga de 15, une teneur pondérale en sodium par rapport au poids de zéolithe oméga sèche de 160 ppm, un volume de maille élémentaire de 2,145 nm³, et une capacité d'adsorption en azote mesurée à -196°C et à P/Pₒ=0,19 de 0,214 cm³ liquide/g.

La zéolithe oméga, précédemment obtenue, est ensuite soumise au traitement par une solution d'hexafluorosilicate d'ammonium. Pour cela, 20 grammes de zéolithe sèche sont mises en suspension dans 200 ml d'une solution d'acétate d'ammonium (25 grammes d'acétate d'ammonium pour 200 ml d'eau distillée). Cette suspension est ensuite placée dans un tricol de 500 ml équipé d'un reflux, et d'une agitation mécanique, le pH initial du milieu est de 6,9. La température est portée à 80°C. Puis à l'aide d'une pompe, 95 ml d'une solution d'hexafluorosilicate d'ammonium 0,5 M sont introduits à la vitesse de 20 ml/h par gramme de zéolithe sèche traitée, soit un débit de solution de 6,67 ml/min. Finalement, la quantité d'hexafluorosilicate d'ammonium injectée représente 1,71 moles d'hexafluorosilicate d'ammonium par mole d'aluminium présente dans la zéolithe oméga séche. Le système est maintenu à la température de la réaction durant encore 2 heures. Puis, la solution est refroidie à l'ambiante, la valeur mesurée du pH en fin de réaction est de 5,8. Le solide est alors filtré et lavé avec un volume d'eau distillée bouillante au moins égal à 4 litres, c'est à dire au moins 200 ml d'eau distillée par gramme de zéolithe sèche (V/P=200 ml/g). La zéolithe ainsi traitée est séchée à l'étuve à 105°C durant une nuit, puis calcinée sous air sec de manière à déammoniaquer la zéolithe oméga et à obtenir la forme H. Le solide obtenu à l'issue des ces traitements est référencé Ω1. Cette zéolithe oméga, Ω1, posséde un rapport Si/Al global, détermine par fluorescence X de 15,8 et un rapport Si/Al, déterminé par XPS, sur la surface externe des cristaux de la zéolithe, de 43.

### Exemple 2 : Préparation du catalyseur C1 selon l'invention

La zéolithe Ω1 obtenue à l'exemple 1 est ensuite mise en forme par extrusion avec un gel d'alumine de manière à obtenir, après séchage et calcination sous air sec, le catalyseur **C1** qui contient en % poids 80 % de zéolithe oméga et 20 % d'alumine.

### Exemple 3 : Préparation du catalyseur C2 selon l'invention

Dans cet exemple, le catalyseur C1 préparé dans l'exemple 2, est soumis à 3 échanges ioniques avec une solution d'acétate de nickel de manière à introduire 1,0% poids de nickel dans le catalyseur.

Pour cela, le catalyseur C1 est mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est pour chaque échange ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur **C2** ainsi obtenu contient, en % poids, 79,30 % de zéolithe oméga forme hydrogène, 19,80 % d'alumine et 0,85 % de nickel.

### Exemple 4 : Préparation du catalyseur C3 non conforme

La matière première utilisée est une zéolithe oméga, qui possède un rapport atomique Si/Al global égal à 3,2, une teneur pondérale en sodium par rapport au poids en zéolithe oméga sèche d'environ 5,3 %, un volume de maille élémentaire de 2,196 nm³, et un volume poreux, à l'azote, mesuré à -196°C et à P/Pₒ=0,19 de 0,125 cm³ liquide par gramme.

Cette zéolithe oméga subit tout d'abord une calcination dite sèche à 550°C sous flux d'air et d'azote durant 6 heures. Puis, le solide obtenu est soumis à trois échanges ioniques dans une solution de NH₄NO₃ 10N, à environ 100°C pendant 4 heures pour chaque échange. La zéolithe oméga est alors soumise à un traitement hydrothermique, en présence de 50% de vapeur d'eau à 625°C, durant 4 heures. La zéolithe subit alors une attaque acide, à l'aide d'acide nitrique 1,5N, à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10).

A l'issue de ces traitements, la zéolithe oméga sous forme H a un rapport Si/Al atomique global égal à 11,3, un rapport Si/Al de charpente, déterminé par RMN du ²⁹Si de 12, une teneur pondérale en sodium par rapport au poids de zéolithe oméga sèche de 160 ppm, un volume de maille élémentaire de 2,145 nm³, et une capacité d'adsorption en azote mesurée à -196°C et à P/Pₒ=0,19 de 0,214 cm³ liquide/g.

La zéolithe précédemment obtenue est ensuite mise en forme par extrusion avec un gel d'alumine de manière à obtenir, après séchage et calcination sous air sec, le catalyseur **C3** qui contient en poids 80 % de zéolithe oméga et 20 % d'alumine.

### Exemple 5 : Préparation du catalyseur C4 non conforme

Dans cet exemple, le catalyseur C3 préparé dans l'exemple 4, est soumis à 3 échanges ioniques avec une solution d'acétate de nickel de manière à introduire 1 % poids de nickel dans le catalyseur.

Pour cela, le catalyseur C3 est mis en contact avec une solution 0.5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est pour chaque échange ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur **C4** ainsi obtenu contient, en % poids, 79,30 % de zéolithe oméga forme hydrogène, 19,80 % d'alumine et 0,85 % de Nickel.

### Exemple 6 : Evaluation des performances des catalyseurs

Les catalyseurs sont mis en oeuvre dans un réacteur à lit fixe, sous pression, dans lequel est introduit la charge qui est constituée de toluène pur.

La comparaison des rendements en (benzène + ethylbenzène + xylènes) obtenus en utilisant les catalyseurs C1 et C2, conformes à l'invention, et C3 et C4, non conformes à l'invention, est effectuée dans le tableau ci-après :

| Catalyseurs | C1 (conforme) | C3 (non conforme) | C2 (conforme) | C4 (non conforme) |
|---|---|---|---|---|
| Température de réaction (°C) | 450 | 450 | 430 | 430 |
| Pression totale de réaction (Bar) | 30 | 30 | 40 | 40 |
| Rendements % poids (Benzène + Ethylbenzène + xylènes) | 38,6 | 38,0 | 37,1 | 36,3 |

La comparaison des catalyseurs C1 et C3 et d'autre part la comparaison des catalyseurs C2 et C4, montre que les catalyseurs selon l'invention, C1 et C2, conduisent à des rendements en (Benzène + Ethylbenzène + xylènes) supérieurs à ceux obtenus avec les catalyseurs non conformes C3 et C4.

## Revendications

1. Catalyseur comportant au moins une matrice et au moins une zéolithe de type structural mazzite, au moins en partie sous forme acide, comprenant du silicium et de l'aluminium, ayant été préparée par au moins un traitement par au moins une solution d'un fluorosilicate d'un cation à raison de 0,05 à 5 moles par mole d'aluminium contenue dans la zéolithe sèche, ledit catalyseur comprenant éventuellement au moins un élément choisi dans l'ensemble formé par les groupes IB et VIII de la classification périodique des éléments

2. Catalyseur selon la revendication 1 tel que ladite zéolithe est choisie dans le groupe formé par la zéolithe mazzite gallosilicate, la mazzite, la zéolithe LZ-202, la zéolithe oméga ou la zéolithe ZSM-4.

3. Catalyseur selon l'une des revendications 1 ou 2 tel que ladite zéolithe est la zéolithe oméga.

4. Catalyseur selon l'une des revendications 1 à 4 tel que la zéolithe a été préparée à partir d'un sel de fluorosilicate choisi parmi les sels possédant la formule suivante: M_{2/x}SiF₆, où M est un cation métallique ou non métallique possédant la valence x.

5. Catalyseur selon la revendication 4 tel que le cation M est choisi dans le groupe formé par NH₄⁺, des alkyls ammonium, K⁺, Na⁺, Li⁺, Ba²⁺, Mg²⁺, Cd²⁺, Cu⁺, Cu²⁺, Ca²⁺, Cs⁺, Fe²⁺, Co²⁺, Pb²⁺, Mn²⁺, Rb⁺, Ag⁺, Sr²⁺, Zn²⁺, Tl⁺ et H⁺.

6. Catalyseur selon l'une des revendications 4 ou 5 tel que le sel d'hexafluorosilicate est l'hexafluorosilicate d'ammonium.

7. Catalyseur selon l'une des revendications 1 à 6 tel que ledit élément est choisi dans le groupe formé par Ag, Pt et Ni.

8. Catalyseur selon l'une des revendications 1 à 7 comprenant de 10 à 99% de zéolithe et, dans le cas où ledit catalyseur contient au moins un élément choisi dans l'ensemble formé par les groupes IB et VIII de la classification périodique des éléments, entre 0,01 et 10% dudit élément, le complément à 100 % poids consistant en la part de matrice du catalyseur.

9. Catalyseur selon l'une des revendications 1 à 8 sous forme de pastilles, d'aggrégats, d'extrudés ou de billes.

10. Préparation du catalyseur selon l'une des revendications 1 à 9 comportant le mélange de la matrice et de la zéolithe puis la mise en forme, ainsi que le dépôt éventuel dudit élément.

11. Préparation du catalyseur selon la revendication 10 comportant une calcination finale à une température comprise entre 250 et 600 °C.

12. Utilisation du catalyseur selon l'une des revendications 1 à 9 ou préparé selon l'une des revendications 10 ou 11 en dismutation d'hydrocarbures alkylaromatiques et/ou en transalkylation d'hydrocarbures alkylaromatiques.

13. Utilisation selon la revendication 12 en dismutation et/ou en transalkylation de toluène et/ou d'alkylaromatiques à au moins 9 atomes de carbone par molécule.
